Europäisches Patentamt

European Patent Office

Office européen des brevets

(11) Publication number: **0 208 520 B1**

## EUROPEAN PATENT SPECIFICATION

(45) Date of publication of patent specification: **15.04.92**

(51) Int. Cl.[5]: **A61B 5/02**

(21) Application number: **86305197.5**

(22) Date of filing: **04.07.86**

---

(54) **Oscillometric blood pressure monitor employing non uniform pressure decrementing steps.**

---

(30) Priority: **05.07.85 US 751840**

(43) Date of publication of application:
**14.01.87 Bulletin 87/03**

(45) Publication of the grant of the patent:
**15.04.92 Bulletin 92/16**

(84) Designated Contracting States:
**AT BE CH DE FR GB IT LI LU NL SE**

(56) References cited:
EP-A- 0 020 110      EP-A- 0 079 305
DE-A- 3 424 535      US-A- 4 116 230
US-A- 4 271 843      US-A- 4 360 029

(73) Proprietor: **CRITIKON, INC.**
**4110 George Road**
**Tampa Florida 33607(US)**

(72) Inventor: **Ramsey III, Maynard**
**903 Golfview**
**Tampa, FL 33609(US)**
Inventor: **Medero, Richard**
**2410 Blindpond Avenue**
**Lutz, FL 33549(US)**
Inventor: **Hood, Rush W., Jr.**
**15615 Gardenside Lane**
**Tampa, FL 33624(US)**

(74) Representative: **Jones, Alan John et al**
**CARPMAELS & RANSFORD 43 Bloomsbury**
**Square**
**London, WC1A 2RA(GB)**

---

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to automated blood pressure monitoring, and more particularly to that class of automated blood pressure monitors that utilize a pneumatic cuff for accomplishing a sphygmomanometric measurement on a subject.

Reference is hereby made to the following concurrently filed co-pending European patent applications:

IMPROVED SPHYGMOMANOMETRIC CUFF PRESSURIZING SYSTEM, EP-A-0 207 805;

IMPROVED AUTOMATED MEAN ARTERIAL BLOOD PRESSURE MONITOR WITH DATA ENHANCEMENT, EP-A-0 208 521;

IMPROVED AUTOMATED SYSTOLIC BLOOD PRESSURE MONITOR WITH DATA ENHANCEMENT, EP-A-0 207 806; and

IMPROVED AUTOMATED DIASTOLIC BLOOD PRESSURE MONITOR WITH DATA ENHANCEMENT, EP-A-0 207 807.

The sphygmomanometric class of automated blood pressure monitors employ an inflatable cuff to exert controlled counter-pressure on the vasculature of the subject. One large class of such monitors, exemplified by that described in US-4,349,034 and US-4,360,029 employs the oscillometric methodology. In accordance with this methodology, an inflatable cuff is suitably located on the limb of a patient and is pumped up to a predetermined pressure. Thereupon, the cuff pressure is reduced in predetermined decrements, and at each level pressure fluctuations are monitored. The resultant signals typically consist of the DC voltage with a small superimposed variational component caused by arterial blood pressure pulsations (referred to herein as "oscillatory complexes" or just simply "oscillations"). After suitable filtering to reject the DC component and to provide amplification, peak pulse amplitudes (PPA) above a given base-line are measured and stored. As the decrementing continues, the peak amplitudes will normally increase from a lower level to a relative maximum, and thereafter will decrease. The lowest cuff pressure at which the oscillations have a maximum value is representative of mean arterial pressure. Systolic and diastolic pressures can be derived either as predetermined fractions of mean arterial pressure, or by more sophisticated methods of direct processing of the oscillatory complexes.

The step deflation technique as set forth in this methodology has become the commercial standard of operation. A large percentage of clinically acceptable automated blood pressure monitors utilize the step deflation rationale, and although development efforts have been directed to continuous deflate monitors, substantial difficulties have been encountered in securing accurate and reliable clinical results. Indeed, at least one commercial blood pressure system which feature the continuous deflate mode also employs a step deflation backup system, which is utilized to insure accurate results for those circumstances in which the continuous deflation proves inadequate. DE-A-3,424,535, which is a National German application published after the priority date of the present application but has a filing date prior to the priority date of the present application, utilizes continuous deflate mode but also includes steps in the systolic and diastolic pressure regions to obtain accurate results. Thus, while efforts continue unabated for more rapid detection methods which avoid step deflations, the incremental deflate class of instrument enjoys substantial preference among clinicians.

In contemporaneously filed European patent application, EP-A-0 207 805, there is disclosed an apparatus for shortening the time required to inflate the pressure cuff to a level above the systolic pressure of the patient in preparation for deflation and a measurement cycle.

Step deflation measurements as heretofore obtained and as exemplified by US-4 349 034 and US-4 360 029 can require at least 30 seconds to perform and occasionally as much as a full minute. The American Heart Association recommends a deflation rate for manual sphygmomanometric measurement no greater than 267-533 Pa (2-4 Torr) per heart beat. For normal blood pressure measurements this results in manual determination times on the order of 30 seconds. Similarly, with conventional automated noninvasive pressure measuring devices, the time required for a normal determination is on the order of 35 seconds when the deflation steps size is the standard 666.5 to 800 Pa (5 to 6 Torr).

EP-A-0079305 describes two-stage systolic and diastolic blood pressure measurement in which Korotkoff sounds are detected during intermittent deflation of a blood pressure cuff. Systolic and diastolic pressures are determined on the basis of the presence or absence of the Korotkoff sounds.

It is, accordingly, a primary object of the present invention to reduce the overall blood pressure measurement time by reducing the time required for cuff deflation.

It is a further object of the present invention to provide apparatus for obtaining accurate blood pressure measurement while employing significantly larger decremental steps than heretofore thought feasible over a significant portion of the deflation phase of the measurement cycle.

It is a further object of the present invention to provide apparatus capable of large decremental deflation steps within a sufficiently short interval of time to avoid skipping heart beats and thereby prolonging the measurement phase. That is, it is an object of the present invention to operate the deflation cycle at a suitable rate and tempo to coordinate with pulse rates as rapid as 100 per minute.

It is yet another object of the present invention to alleviate patient discomfort by minimizing the amount of time at which the blood pressure cuff is at higher and less comfortable pressure.

In accordance with one aspect of the invention, there is provided an automated sphygmomanometric apparatus comprising in combination: an inflatable and deflatable pressure cuff; inflating means operatively coupled to said cuff for selectably supplying a gaseous medium under pressure to said cuff to inflate and pressurize said cuff; cuff pressure sensing means coupled to said cuff for sensing cuff pressure and any oscillations in cuff pressure; deflate valve means coupled to said cuff for selectably releasing said gaseous medium from said cuff in successive decrements; and processing means responsively coupled to said cuff pressure sensing means for blood pressure related measurements; characterised in that there is provided control means responsive to pressure sensed by said cuff pressure sensing means for automatically establishing, for a next subsequent cuff pressure decrement, as a function of the prevailing cuff pressure, at least one of (i) cuff pressure decrement size and (ii) the rate of release of said gaseous medium, said control means in combination with said deflate valve means being constructed and arranged to deflate said cuff in non-uniform pressure decrementing steps.

In accordance with another aspect of the present invention there is provided deflate valve means comprising means for providing at least two different pressure dependent flow rates, one faster and one slower for any given cuff pressure, for releasing the gaseous inflating medium from the cuff, and control means including rate determining means for ascertaining the rate of deflation prevailing during each successive decrement step, in combination with selector means for initiating release of the gaseous medium from the cuff using the deflate valve means that provides the slower flow rate and continuing to use said slower-flow-rate-providing deflate valve means until the deflation rate has slowed to a predetermined value whereupon the selector means uses the faster-flow-rate-providing deflate valve means, or both valves together.

The invention will be better understood after reading the following detailed description of the presently preferred embodiment thereof with reference to the appended drawings in which:

Fig. 1 is a schematic representation of a system and the basic components embodying the present invention;

Fig. 2 is a flow chart representing the operation of the apparatus of Fig. 1 under the control of the microprocessor or equivalent controller; and

Fig. 3 is a pressure versus time graph comparing the operation of a prior art system with the operation of the present invention.

The same reference numerals are used throughout the drawings to designate the same or similar parts.

Reference should be had to US-4,360,029 which discloses in great detail a system for oscillometric blood pressure monitoring to which the principles of the present invention may be applied with advantage. The disclosure of US-4 360 029 is incorporated by reference herein.

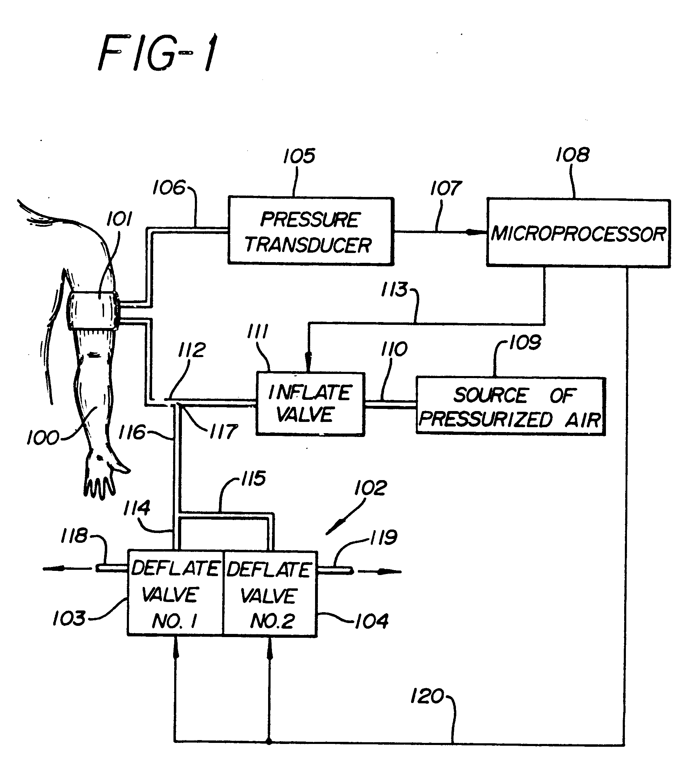

Referring to Fig. 1 herein, there is shown an illustrative embodiment of the principles of the present invention. The arm 100 of a human subject is shown wearing a conventional flexible inflatable and deflatable cuff 101 for occluding the brachial artery when fully inflated. As the cuff 101 is deflated, in a manner to be described further below, via air venting deflate valve apparatus 102 consisting of first and second deflate valves 103 and 104, the arterial occlusion is gradually relieved. A pressure transducer 105 is coupled by a duct 106 to the cuff 101 and senses the pressure therein. In accordance with conventional oscillometric techniques, pressure oscillations in the artery are sensed by changes in the counterpressure of the cuff 101, and in turn by the transducer 105, there to be converted to an electrical signal and coupled over path 107 to a microprocessor or other controller 108. From the standpoint of the principles of the present invention, the processing of the signals from pressure transducer 105 by the microprocessor 108 to produce blood pressure data, and optionally to reject artifact data, can be conducted in accordance with the prior art, for example in accordance with the teachings of US-4 360 029. Alternatively, the blood pressure can be determined in accordance with the teachings of the contemporaneously filed European patent applications, EP-A-0 208 521, EP-A-0 207 806 and EP-A- 207 807.

A source of pressurized air 109 is shown connected via a duct 110 through an inflate valve 111 and a duct 112 to the pressure cuff 101. The inflate valve 111 is electrically controlled through a connection 113 from the microprocessor 108.

The deflate valve apparatus 102 has its valves 103 and 104 connected by respective ducts 114 and 115 to a junction with duct 116 which, in turn, connects to a branch connection at 117 with the duct 112 leading to cuff 101. Exhaust connections from deflate valves 103 and 104 are shown, respectively, at 118 and 119. The valves 103 and 104 receive electrical control over a path 120 from the microprocessor 108.

The apparatus disclosed above with reference to Fig. 1, except for the plural deflate valves 103 and 104 and the programming of the microprocessor 108 herein, can be substantially the same as that disclosed in EP-A-0 207 805, mentioned above. The structure disclosed in said application incorporates a single deflate valve while, as mentioned previously, the subject embodiment has two valves, 103 and 104, which valves preferably, but not necessarily, differ from one another with regard to orifice size. By way of example, valve 103 has a first size orifice and valve 104 has a larger size orifice, each valve being electrically actuatable and having a given finite response time under the control of microprocessor 108. The details of the microprocessor not discussed in EP-A-0 207 805 but necessary for the present invention will be apparent from the following discussion of the operation of the apparatus as disclosed herein.

Referring now to the operation of the apparatus illustrated in Fig. 1, it can be assumed that air under pressure of about 55-69 kPa (8-10 p.s.i.) is available in the source of pressurized air 109. When it is desired to initiate a determination of blood pressure, the microprocessor 108 furnishes a signal over path 113 to open the inflate valve 111. It is assumed that the deflate valve apparatus 102 is closed. Air from the source 109 is communicated through valve 111 and duct 112 to inflate the cuff 101 to a desired level. Preferably, the microprocessor 108 responds to the signal from the pressure transducer 105, indicative of the instantaneous pressure in the cuff 101, to interrupt the inflation of the cuff 101 when the pressure in the cuff reaches a predetermined value above estimated systolic pressure. Such interruption will be accomplished by feeding a signal over path 113 to close inflate valve 111. Once valve 111 has been closed, the blood pressure measurement can be obtained by commencing the deflate routing.

Actual measurement of the blood pressure under the control of the microprocessor 108 and the deflate valve apparatus 102 and as sensed by pressure transducer 105 can be accomplished in any suitable manner such as that disclosed in US-4 349 034 and US-4 360 029 or EP-A-0 208 521. At the completion of each measurement cycle, the deflate valve apparatus 102 can be re-opened as explained hereinafter long enough to relax the cuff pressure substantially completely. Thereafter, the deflate valve apparatus 102 can be closed at the start of a new measurement cycle.

By way of summation, when a blood pressure measurement is desired, the inflate valve will be opened while the cuff pressure is supervised until the cuff pressure reaches the desired level at which time the inflate valve will be closed. Thereafter, the deflate valves are operated and the measurement taken. The operation of the apparatus that has been discussed to this point can be substantially the same as that described in EP-A-0 207 805. The present invention relates to the deflation phase and that operation will now be described.

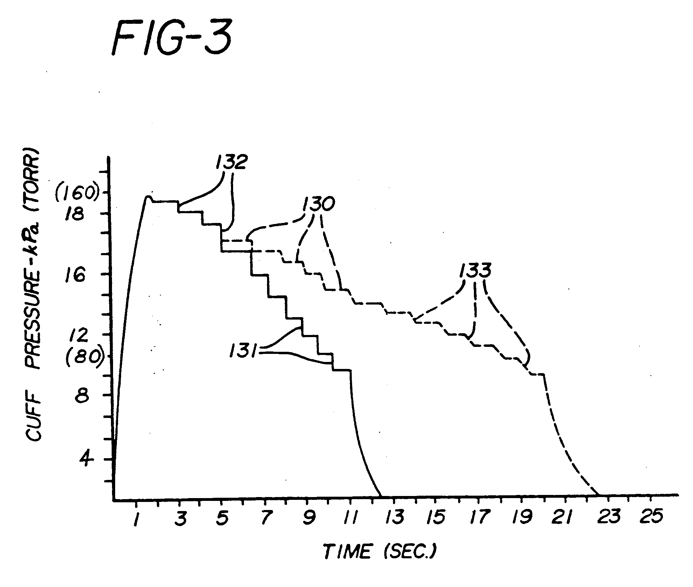

Typically, in prior art automatic sphygmomanometric devices, the cuff deflation operation has been accomplished in equal decremental steps, usually about 666.5 to 800 Pa (5 to 6 Torr), and invariably less than 933 Pa (7 Torr) per step. However, it has now been discovered that reliable and accurate measurements can be obtained even though, contrary to long accepted precepts, steps substantially larger than 933 Pa (7 Torr) are taken, and even though successive steps are of unequal magnitude. Consequently, in accordance with the present invention, the cuff deflation-measurement procedure is accelerated with a resultant significant reduction in overall cycle time. This is illustrated dramatically in Fig. 3 wherein the plot 130 shows that with equal size decrements on the order of 933 Pa (7 Torr) per step, a complete cycle takes about 23 seconds. By contrast, the plot 131, representing operation of the apparatus embodying the present invention, shows completion of a full measuring cycle in less than 13 seconds. While the two plots 130 and 131 represent ideal cases wherein artifact has not interefered with and prolonged the measurement cycle, the plots do reveal the relative time acceleration that can be expected.

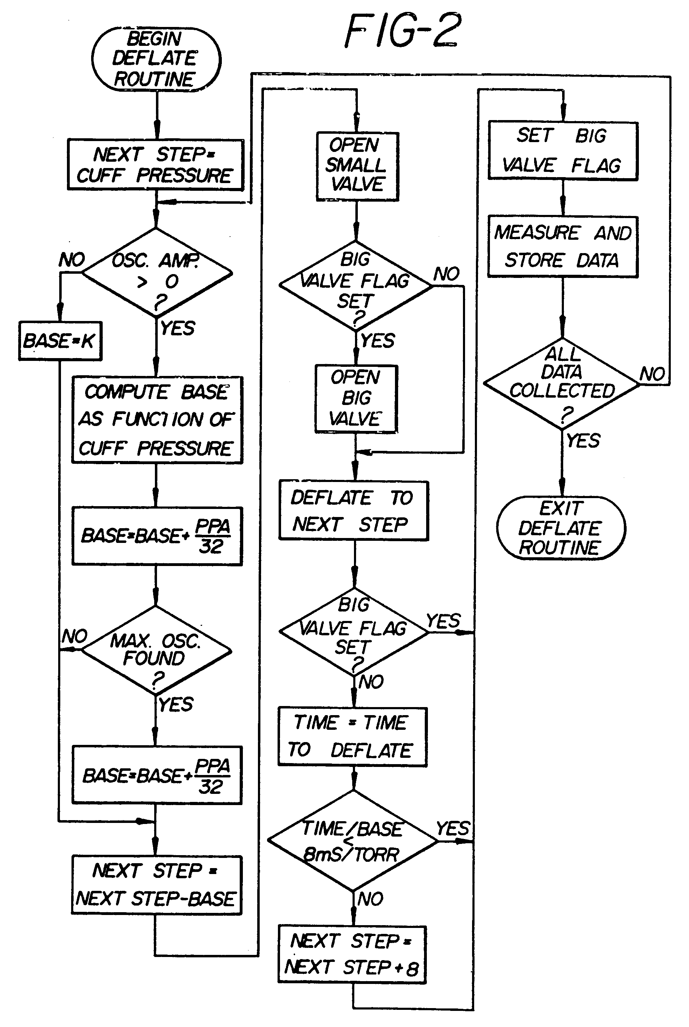

The principles underlying the operation of the present invention are best described with reference to the flow chart in Fig. 2 to which attention should now be directed. At the commencement of the deflation operation or routine, the cuff is deflated by steps of predetermined fixed magnitude, generally between 666.5 and 933 Pa (5 and 7 Torr) per step, until oscillations are detected and validated for the first time. The present example employs steps of 933 Pa (7 Torr) each. Bearing in mind that the cuff pressure is at an upper level, the valve 103 with the smaller orifice is initially employed. For various reasons the subject apparatus is usually employed with the transducer 105 located up to 15 feet or more away from the cuff 101. This distance over which cuff pressure must be conducted via duct 106, as well as the inherent electromechanical limitations of the commonly used deflate valves, introduces a significant response time factor into the activation of the deflate valve apparatus 102. Therefore, the orifice of valve 103 must be small enough that the valve can be opened, cause a desired cuff pressure decrement, and be re-closed before a cuff pressure drop overshoot has occurred.

So long as the cuff pressure is relatively high, the deflation velocity through the smaller orifice valve 103 will be high, and the time required to decrement the pressure the desired step will be relatively short. This is reflected by the comparatively steep or substantially vertical step decrements 132 at the commencement of plots 130 and 131.

If the remaining deflation were to be accomplished only with valve 103 and with equal steps of 933 Pa (7 Torr) each, the time for each decrement would increase, with each successive decrement, (because it occurs at a lower average pressure), and hence take longer and longer. This is represented by the "risers" 133 in plot 130 departing further and further from vertical as deflation progresses. The delay in the measurement is actually aggravated by the lengthened decrement interval because beyond a certain time interval the cuff will still be deflating when the next heart oscillation occurs and such oscillation will have to be skipped by the measuring apparatus, thus requiring a longer period of sampling at that cuff pressure.

The present invention avoids the above mentioned problem by timing each decrement step and by switching over to a larger deflate valve orifice whenever the decrement step requires more than 8 milliseconds to deflate 133 Pa (1 Torr). This is equivalent to a deflation rate of 16.66 kPa (125 Torr) per second. Thus, during a deflation routine, the present apparatus will at some point switch from deflate valve 103 to 104, and, if necessary, make a further switch to operation of both valves 103 and 104 in parallel. A typical deflation rate at the beginning of the deflation operation is about 266.6 kPa (200 Torr) per second. This is equivalent to 37.5 $\mu$s/Pa (5 mSec. per Torr).

As mentioned above and indicated by the flow chart in Fig. 2, after arterial oscillations are detected and verified, i.e., oscillation amplitude is greater than 0, the microprocessor 108 resorts either to a look-up table or to an equivalent formula to select a "Base" deflate step as a function of the then prevailing cuff pressure sensed by transducer 105. A typical table relating "Base" deflate step to cuff pressure can be constructed in the manner described below. Generally, the "Base" deflate steps over the middle range of the deflation procedure are each substantially greater than 933 Pa (7 Torr) and can be as much as 2.67 kPa (20 Torr) or more, particularly when measurements are being made on a subject with excessively high blood pressure.

The flow chart shows, however, an augmentation of the "Base" deflate step using the equation:

Base = Base + PPA/32     (1)

wherein PPA is a quantity directly proportional to the last detected oscillation peak amplitude. For convenience, PPA can be replaced by "x", and for the purpose of generalization, the divisor "32", a constant unique to one specific embodiment, can be represented by the constant "a". The augmentation represented by equation (1) is used in the deflation routing until the microprocessor 108 has detected the maximum amplitude oscillation from the arterial complexes. After detection and verification of the maximum amplitude oscillation, the decrement equation can be further augmented to:

Base = Base + PPA/32 + PPA/32 = Base + 2-(PPA/32).     (2)

Operation in accordance with equations (1) and (2) therefore can be generalized as follows:

Base = Base + y(x/a)     (3)

where "Base" and "x" are as defined previously, "y" is equal to one or two, and "a" is a constant chosen such that the value of "x/a" over the normal range of oscillation amplitudes will vary between zero and about 3.

During each decrement step, a determination is made of the time required to accomplish the decrement and this time is stored to be used during the next decrement procedure. Also stored is the last "Base". By obtaining the quotient of the two stored quantities (Time/Base) and comparing with the preselected rate of 60$\mu$s/Pa (8 mSec./Torr), a determination is made whether to use the same (i.e. smaller) deflate valve for the decrement in process or to also use the larger orifice deflate valve in combination with the smaller valves. The drawings described this particular scheme of operation for the valves.

The need for augmenting the cuff pressure dependent "Base" step by an oscillation amplitude dependent factor is due to two phenomena. First, there can occur a large increase in cuff pressure at each heart beat resulting from arm expansion momentarily during cardiac systole. Second, after the cuff pressure had decreased below that at maximum oscillation amplitude, the blood flow passing under the cuff 101 with each cardiac systole begins to engage the lower arm, which, in turn, causes the pressure in the cuff to slowly rise. The net effect of the two phenomena is to require additional decrement steps in cuff pressure to deflate the cuff below the diastolic pressure level unless the deflate step sizes are increased commensurately with the two phenomena just described.

A look up table relating the "Base" deflate steps to prevailing cuff pressure can be constructed arbitrarily on a point by point basis or using the following type of equation:

Base Step = k*CP     (4)

where "CP" = cuff pressure in Torr (Pa/133.3) and "k" is a constant on the order of 0.1, for example.

Alternatively, when it is desired to place a constraint on the minimum size step, the equation can take the form:

$$\text{Base step} = k_1 + k_2 \cdot CP \qquad (5)$$

where "$k_1$" and "$k_2$" are constants, respectively, on the order of 4 Torr (0.533 kPa) and 0.05, for example, and "CP" is as defined above.

It should be understood that equations such as (4) and (5) can be used directly to compute the values of Base Step as required during a measurement procedure instead of providing a pre-calculated look up table. It should also be understood that the value selected for "a" will depend upon the proportionality factor between "PPA" and actual oscillation peak amplitude.

Referring to the flow chart of Fig. 2, it will be noted that when the 60μs/Pa (8 mSec./Torr) decrement interval is exceeded, the NEXT STEP value (i.e., the desired new cuff pressure) is increased by "8". This is to ensure against overshooting the desired pressure level when first using the larger valve.

While valves 103 and 104 have been described as having different size orifices, it is contemplated that equal size valves can be used. In such case, the operating routine would be arranged to commence the deflate cycle using one valve, with a switch to two valves in parallel when an increased flow rate for the particular pressure level is desired. Another alternative would be to have a controllable throttling valve operable between two or more orifice settings. In any event, the deflate valve mechanism should have at least two operating modes, one providing a greater flow rate than the other for any given applied pressure.

## Claims

1. Automated sphygmomanometric apparatus comprising in combination:
   a) an inflatable and deflatable pressure cuff (101);
   b) inflating means operatively coupled to said cuff for selectably supplying a gaseous medium under pressure to said cuff to inflate and pressurize said cuff;
   c) cuff pressure sensing means (105) coupled to said cuff for sensing cuff pressure and any oscillations in cuff pressure;
   d) deflate valve means (102) coupled to said cuff for selectably releasing said gaseous medium from said cuff in successive decrements; and

   e) processing means (108) responsively coupled to said cuff pressure sensing means, for blood pressure related measurements;
   characterised in that there is provided
   (f) control means (108) responsive to pressure sensed by said cuff pressure sensing means for automatically establishing, for a next subsequent cuff pressure decrement, as a function of the prevailing cuff pressure, at least one of
      (i) cuff pressure decrement size and
      (ii) the rate of release of said gaseous medium, said control means in combination with said deflate valve means being constructed and arranged to deflate said cuff in non-uniform pressure decrementing steps.

2. Automated sphygmomanometric apparatus according to claim 1, characterised in that said deflate valve means comprise means for providing at least two different pressure dependent flow rates, one faster and one slower for any given cuff pressure, for releasing said gaseous medium from said cuff, and said control means comprises selector means for initiating release of said gaseous medium from said cuff using said deflate valve means that provides the slower flow rate (103) and continuing to use said slower-flow-rate-providing deflate valve means (103) until the deflation rate has slowed to a predetermined value whereupon said selector means uses said faster-flow-rate-providing deflate valve means (104), or both valve means together.

3. Automated sphygmomanometric apparatus according to claim 2, wherein said control means includes rate determining means for ascertaining the rate of deflation prevailing during each successive decrement step.

4. Automated sphygmomanometric apparatus according to claim 2 or claim 3, characterized in that said deflate valve means comprises a first valve (103) with a given size orifice, and a second valve (104) with an orifice larger than said given size orifice; and said selector means is constructed and arranged to commence a deflation cycle using said first valve, and to switch to said second valve if the deflation rate with said first valve is slower than said predetermined value.

5. Automated sphygmomanometric apparatus according to claim 4, characterised in that said selector means is further constructed and arranged to switch to parallel use of said first and second valves whenever said flow rate using said second valve becomes slower than said predetermined value, until said cuff has attained desired deflation.

6. Automated sphygmomanometric apparatus according to claim 2 or claim 3 characterised in that said deflate valve means comprises two valves each with the same size orifice; and said selector means is constructed and arranged to commence a deflation cycle using only one of said deflate valves, and to switch to parallel use of both of said two valves if the deflation rate with said one deflate valve is slower than said predetermined value.

7. Automated sphygmomanometric apparatus according to any one of claims 1 to 6, characterised in that said control means comprises deflate step determining means for causing substantially equal successive pressure decrements after initiation of a cuff deflation procedure and before an arterial pressure oscillation is detected by said processing means and causing increased decrement steps after said oscillation is detected.

8. Automated sphygmomanometric apparatus according to claim 7, characterised in that said step determining means is constructed and arranged to cause said increased decrement steps in accordance with the relationship:

Decrement Step = Base Step + y (x/a)

wherein Base Step is a predetermined function of prevailing cuff pressure, "x" is a quantity proportional to the last detected oscillation amplitude, "y" is equal to one or two, and "a" is a constant.

9. Automated sphygmomanometric apparatus according to claim 8, characterised in that "y" is equal to one during a measurement cycle until a maximum amplitude oscillation has been detected and identified.

10. Automated sphygmomanometric apparatus according to any one of claims 1 to 6, characterised in that said control means comprises deflate step determining means coupled to said cuff pressure sensing means for causing said pressure decrementing steps to vary in magnitude as a function of the prevailing cuff pressure.

11. Automated sphygmomanometric apparatus according to claim 10, characterised in that said pressure decrementing steps are determined, at least in part, so as to satisfy the equation:

Decrementing Step = k*CP

where "CP" = cuff pressure and "k" is a constant having a value on the order of 0.1.

12. Automated sphygmomanometric apparatus according to claim 10 characterized in that said pressure decrementing steps are determined, at least in part, so as to satisfy the equation:

Decrementing Step = $k_1$ + $k_2$*CP

where "CP" = cuff pressure in Pa and "$k_1$" and "$k_2$" are constants having respective values on the order of 533 Pa (4 Torr) and 0.05.

13. Automated sphygmomanometric apparatus according to any one of claims 7 to 12, characterised in that said deflate step determining means is adapted to cause at least some of said pressure decrementing steps to be in excess of 933 Pa (7 Torr).

14. Automated sphygmomanometric apparatus according to any one of claims 1 to 13, further including processing means responsively coupled to said cuff pressure sensing means for providing blood pressure related measurements.

**Revendications**

1. Appareil sphygmomanométrique automatique comportant en combinaison:
   a) un manchon sous pression (101) gonflable et dégonflable;
   b) des moyens de gonflage fonctionnellement couplés audit manchon pour fournir sélectivement un médium gazeux sous pression audit manchon pour gonfler et mettre sous pression ledit manchon;
   c) des moyens (105) de détection de la pression régnant dans le manchon couplés audit manchon pour détecter la pression régnant dans le manchon et toutes les oscillations de la pression régnant dans le manchon;
   d) des moyens (102) formant vanne de dégonflage couplés audit manchon pour évacuer sélectivement ledit médium gazeux hors dudit manchon par pas décrémentiels successifs; et
   e) des moyens de traitement (108), couplés en y étant sensibles, auxdits moyens de détection de la pression régnant dans le manchon, pour des mesures se rapportant à la pression sanguine;

appareil caractérisé par le fait qu'il y est prévu

f) des moyens de commande (108), sensibles à la pression détectée par lesdits moyens de détection de la pression régnant dans le manchon, pour établir automatiquement, pour un pas décrémentiel, venant immédiatement à la suite, de la pression régnant dans le manchon, comme fonction de la pression régnant actuellement dans le manchon, au moins l'une des deux valeurs suivantes

(i) la valeur du pas décrémentiel de la pression régnant dans le manchon et

(ii) la cadence d'évacuation dudit fluide gazeux, lesdits moyens de commande, en combinaison avec lesdits moyens formant vanne de dégonflage, étant construits et disposés pour dégonfler ledit manchon par pas non uniformes décrémentant la pression.

2. Appareil sphygmomanométrique automatique selon la revendication 1,

caractérisé par le fait que lesdits moyens formant vanne de dégonflage comportent des moyens pour donner au moins deux débits différents, fonction de la pression, un débit plus rapide et un débit plus lent, pour toute pression donnée régnant dans le manchon, pour évacuer ledit médium gazeux hors dudit manchon, et par le fait que lesdits moyens de commande comportent des moyens de sélection pour démarrer l'évacuation dudit fluide gazeux hors dudit manchon en utilisant lesdits moyens formant vanne de dégonflage qui donnent le débit plus lent (103) et pour continuer d'utiliser lesdits moyens (103), formant vanne de dégonflage et donnant le débit plus lent, jusqu'à ce que le débit de dégonflage ait ralenti jusqu'à une valeur prédéterminée, sur quoi lesdits moyens de sélection utilisent lesdits moyens (104) formant vanne de dégonflage donnant le débit plus rapide, ou bien les deux moyens formant vanne, ensemble.

3. Appareil sphygmomanométrique automatique selon la revendication 2, dans lequel lesdits moyens de commande comportent des moyens de déterminer la cadence pour se rendre compte de la cadence de dégonflage en vigueur au cours de chaque pas décrémentiel successif.

4. Appareil sphygmomanométrique automatique selon la revendication 2 ou la revendication 3, caractérisé par le fait que lesdits moyens formant vanne de dégonflage comportent une première vanne (103) avec un orifice de dimension donnée, et une seconde vanne (104) avec un orifice plus grand que ledit orifice de dimension donnée; et par le fait que lesdits moyens de sélection sont construits et disposés pour commencer un cycle de dégonflage en utilisant ladite première vanne puis pour commuter sur ladite seconde vanne si la cadence de dégonflage avec ladite première vanne est inférieure à ladite valeur prédéterminée.

5. Appareil sphygmomanométrique automatique selon la revendication 4, caractérisé par le fait que lesdits moyens de sélection sont en outre construits et disposés pour commuter sur l'emploi, en parallèle, de ladite première et de ladite seconde vannes, chaque fois que ladite cadence, en utilisant ladite seconde vanne, devient plus lente que ladite valeur prédéterminée, jusqu'à ce que ledit manchon ait atteint le dégonflage désiré.

6. Appareil sphygmomanométrique automatique selon la revendication 2 ou la revendication 3, caractérisé par le fait que lesdits moyens formant vanne de dégonflage comportent deux vannes, chacune avec un orifice de la même dimension; et par le fait que lesdits moyens de sélection sont construits et disposés pour commencer un cycle de dégonlfage en n'utilisant qu'une seule desdites vannes de dégonflage, puis pour commuter sur l'emploi en parallèle des deux desdites deux vannes si la cadence de dégonflage avec ladite première vanne de dégonflage est inférieure à ladite valeur prédéterminée.

7. Appareil sphygmomanométrique automatique selon l'une quelconque des revendications 1 à 6, caractérisé par le fait que lesdits moyens de commande comportent des moyens de déterminer le pas décrémentiel de dégonflage pour provoquer des décréments de pression successifs substantiellement égaux après démarrage d'une procédure de dégonflage du manchon et avant qu'une oscillation de la pression artérielle soit détectée par lesdits moyens de traitement et pour provoquer une augmentation susdite du pas décrémentiel après que ladite oscillation est détectée.

8. Appareil sphygmomanométrique automatique selon la revendication 7, caractérisé par le fait que lesdits moyens de détermination des pas décrémentiels sont construits et disposés pour faire en sorte que ladite augmentation du pas décrémentiel se fasse selon la relation:

Pas décrémentiel = pas de base + y (x/a)

où "pas de base" est une fonction prédéterminée de la pression régnant dans le manchon, "x" est une quantité proportionnelle à l'amplitude de la dernière oscillation détectée, "y" est égal à un ou deux, et "a" est une constante.

9. Appareil sphygmomanométrique automatique selon la revendication 8, caractérisé par le fait que "y" est égal à un au cours d'un cycle de mesure jusqu'à ce qu'une oscillation d'amplitude maximale ait été détectée et identifiée.

10. Appareil sphygmomanométrique automatique selon l'une quelconque des revendications 1 à 6, caractérisé par le fait que lesdits moyens de commande comportent des moyens de détermination du pas décrémentiel de dégonflage couplés auxdits moyens de détection de la pression régnant dans le manchon pour faire en sorte que la valeur desdits pas décrémentant la pression varie en fonction de la pression régnant dans le manchon.

11. Appareil sphygmomanométrique automatique selon la revendication 10, caractérisé par le fait que lesdits pas décrémentant la pression sont déterminés, au moins en partie, de façon à satisfaire l'équation:

Pas décrémentiel = k*CP

où "CP" = pression régnant dans le manchon et "k" est une constante dont la valeur est de l'ordre de 0,1.

12. Appareil sphygmomanométrique automatique selon la revendication 10, caractérisé par le fait que lesdits pas décrémentant la pression sont déterminés, au moins en partie, de façon à satisfaire l'équation:

Pas décrémentiel = $k_1$ + $k_2$*CP

où "CP" = pression régnant dans le manchon en Pa et "$k_1$" et "$k_2$" sont des constantes dont les valeurs respectives sont de l'ordre de 533 Pa (4 Torr) et 0,05.

13. Appareil sphygmomanométrique automatique selon l'une des revendications 7 à 12, caractérisé par le fait que lesdits moyens de détermination du pas décrémentiel de dégonflage sont conçus pour faire en sorte qu'au moins certains desdits pas décrémentant la pression soient supérieurs à 933 Pa (7 Torr).

14. Appareil sphygmanométrique selon l'une quelconque des revendications 1 à 13, comportant en outre des moyens de traitement couplés, et sensibles, auxdits moyens de détection de la pression régnant dans le manchon pour fournir des mesures relatives à la pression sanguine.

## Patentansprüche

1. Automatisches Blutdruckmeßgerät, das in Kombination aufweist:
   a) eine aufpumpbare und ablaßbare Druckmanschette (101);
   b) Aufpumpmittel, die betriebsmäßig an die Manschette angeschlossen sind, um der Manschette ein gasförmiges Medium unter Druck wahlweise zuzuführen, damit die Manschette aufgepumpt und unter Druck gesetzt wird;
   c) Manschettendruck-Fühlmittel (105), die an die Manschette angeschlossen sind, um den Manschettendruck und jegliche Schwankungen des Manschettendruckes zu erfassen;
   d) Ablaßventilmittel (102), die an die Manschette angeschlossen sind, um das gasförmige Medium in aufeinanderfolgenden Abnahmeschritten aus der Manschette wahlweise abzulassen; und
   e) Verarbeitungsmittel (108), die an die Manschettendruck-Fühlmittel angeschlossen sind, so daß sie auf diese für blutdruckbezogene Messungen ansprechen,
       dadurch gekennzeichnet, daß
   (f) Steuermittel (108) vorgesehen sind, die auf den von den Manschettendruck-Fühlmitteln erfaßten Druck ansprechen, um für den nächstfolgenden Manschettendruck-Abnahmeschritt in Abhängigkeit des herrschenden Manschettendruckes zumindest einen der folgenden Werte automatisch einzustellen:
       (i) Manschettendruck-Abnahmeschrittgröße und
       (ii) Ablaßgeschwindigkeit des gasförmigen Mediums, wobei die Steuermittel zusammen mit den Ablaßventilmitteln so ausgebildet und angeordnet sind, daß sie die Manschette in ungleichförmigen Druckabnahmeschritten ablassen.

2. Automatisches Blutdruckmeßgerät nach Anspruch 1, dadurch gekennzeichnet, daß die Ablaßventilmittel Mittel zur Schaffung zumindest zweier verschiedener druckabhängiger Strömungsgeschwindigkeiten aufweisen, u.zw. einer schnelleren und einer langsameren für jeden gegebenen Manschettendruck, um das gasförmige Medium aus der Manschette abzulassen, und die Steuermittel Auswahlmittel aufweisen, um das Ablassen des gasförmigen Mediums aus der Manschette unter Verwendung jener Ablaßventilmittel (103) einzuleiten, die die langsamere Strömungsgeschwindigkeit ermöglichen, und um die Verwendung dieser die langsamere Strömungsgeschwindigkeit ermöglichenden Ablaßventilmittel (103) fortzusetzen, bis die Ablaßgeschwindigkeit sich auf einen vorgegebenen Wert verlangsamt hat, woraufhin die Auswahlmittel die die schnellere Strömungsgeschwindigkeit ermöglichenden Ablaßventilmittel (104) oder beide Ventilmittel zusammen einsetzen.

3. Automatisches Blutdruckmeßgerät nach Anspruch 2, bei welchem die Steuermittel Geschwindigkeits-Erfassungsmittel aufweisen, um die während jedes aufeinanderfolgenden Abnahmeschrittes herrschende Ablaßgeschwindigkeit festzustellen.

4. Automatisches Blutdruckmeßgerät nach Anspruch 2 oder 3, dadurch gekennzeichnet, daß die Ablaßventilmittel ein erstes Ventil (103) mit einer Öffnung gegebener Größe und ein zweites Ventil (104) mit einer Öffnung, die größer ist als jene Öffnung gegebener Größe, aufweist; und wobei die Auswahlmittel so ausgebildet und angeordnet sind, daß sie einen Ablaßzyklus unter Verwendung des ersten Ventiles beginnen und auf das zweite Ventil umschalten, wenn die Ablaßgeschwindigkeit mit dem ersten Ventil langsamer ist als ein vorgegebener Wert.

5. Automatisches Blutdruckmeßgerät nach Anspruch 4, dadurch gekennzeichnet, daß die Auswahlmittel weiters so aufgebaut und angeordnet sind, daß sie auf die parallele Verwendung des ersten und des zweiten Ventiles umschalten, wenn die Strömungsgeschwindigkeit unter Verwendung des zweiten Ventiles langsamer wird als der vorgegebene Wert, solange bis die Manschette den gewünschten Ablaßzustand erreicht hat.

6. Automatisches Blutdruckmeßgerät nach Anspruch 2 oder 3, dadurch gekennzeichnet, daß die Ablaßventilmittel zwei Ventile mit jeweils der gleichen Öffnungsgröße aufweisen; und daß die Auswahlmittel so ausgebildet und angeordnet sind, daß sie einen Ablaßzyklus unter Verwendung nur eines der Ablaßventile beginnen und auf die gleichzeitige Verwendung beider Ventile umschalten, wenn die Ablaßgeschwindigkeit mit dem einen Ablaßventil langsamer als der vorgegebene Wert ist.

7. Automatisches Blutdruckmeßgerät nach einem der Ansprüche 1 bis 6, dadurch gekennzeichnet, daß die Steuermittel Ablaßschritt-Bestimmungsmittel aufweisen, um im wesentlichen gleiche aufeinanderfolgende Druckabnahmeschritte nach dem Einleiten eines Manschettenablaßvorganges und vor dem Erkennen einer arteriellen Druckschwankung durch die Verarbeitungsmittel zu veranlassen und nach dem Erkennen der Druckschwankung vorvergrößerte Abnahmeschritte zu veranlassen.

8. Automatisches Blutdruckmeßgerät nach Anspruch 7, dadurch gekennzeichnet, daß die Schritt-Bestimmungsmittel so ausgebildet und angeordnet sind, daß sie die vergrößerten Abnahmeschritte gemäß der Beziehung:

$$Abnahmeschritt = Grundschritt + y (x/a)$$

veranlassen, wobei der Grundschritt eine vorgegebene Funktion des herrschenden Manschettendruckes ist, "x" eine der zuletzt erfaßten Schwankungsamplitude proportionale Größe ist, "y" gleich eins oder zwei und "a" eine Konstante ist.

9. Automatisches Blutdruckmeßgerät nach Anspruch 8, dadurch gekennzeichnet, daß während eines Meßzyklus "y" gleich eins ist, bis eine maximale Amplitudenschwankung erfaßt und identifiziert worden ist.

10. Automatisches Blutdruckmeßgerät nach einem der Ansprüche 1 bis 6, dadurch gekennzeichnet, daß die Steuermittel Ablaßschritt-Bestimmungsmittel aufweisen, die an die Manschettendruck-Fühlmittel angeschlossen sind, um zu bewirken, daß die Druckabnahmeschritte in der Größe als Funktion des herrschenden Manschettendruckes variieren.

11. Automatisches Blutdruckmeßgerät nach Anspruch 10, dadurch gekennzeichnet, daß die Druckabnahmeschritte zumindest teilweise so bestimmt werden, daß sie der Gleichung:

$$Abnahmeschritt = k*CP$$

genügen, worin "CP" = Manschettendruck und "k" eine Konstante mit einem Wert in der Größenordnung von 0,1 ist.

12. Automatisches Blutdruckmeßgerät nach Anspruch 10, dadurch gekennzeichnet, daß die Druckabnahmeschritte zumindest teilweise so bestimmt werden, daß sie der Gleichung

Abnahmeschritt = $k_1$ + $k_2$ * CP

genügen, worin "CP" = Manschettendruck in Pa und "$k_1$" und "$k_2$" Konstanten mit Werten in der Größenordnung von 533 Pa (4 Torr) bzw. 0,05 sind.

13. Automatisches Blutdruckmeßgerät nach einem der Ansprüche 7 bis 12, dadurch gekennzeichnet, daß die Ablaßschritt-Bestimmungsmittel dazu eingerichtet sind, zu bewirken, daß zumindest einige der Druckabnahmeschritte über 933 Pa (7 Torr) liegen.

14. Automatisches Blutdruckmeßgerät nach einem der Ansprüche 1 bis 13, welches ferner Verarbeitungsmittel aufweist, die an die Manschettendruck-Fühlmittel angeschlossen sind, so daß sie auf diese ansprechen, um blutdruckbezogene Messungen zu ermöglichen.

# FIG-1

# FIG-2

```
                    ┌──────────┐
                   ( BEGIN     )
                   ( DEFLATE   )
                   ( ROUTINE   )
                    └────┬─────┘
                         │
              ┌──────────▼──────────┐
              │ NEXT STEP =         │
              │ CUFF PRESSURE       │
              └──────────┬──────────┘
                         │
        NO          ◇ OSC. AMP. ◇
       ┌───────────◇   > O      ◇
       │           ◇    ?       ◇
       │                │ YES
  ┌────▼────┐           │
  │ BASE=K  │  ┌────────▼─────────┐
  └────┬────┘  │ COMPUTE BASE     │
       │       │ AS FUNCTION OF   │
       │       │ CUFF PRESSURE    │
       │       └────────┬─────────┘
       │                │
       │       ┌────────▼─────────┐
       │       │ BASE=BASE+ PPA/32│
       │       └────────┬─────────┘
       │                │
       │      NO    ◇ MAX. OSC. ◇
       │     ┌─────◇   FOUND    ◇
       │     │     ◇     ?      ◇
       │     │          │ YES
       │     │  ┌───────▼──────────┐
       │     │  │ BASE=BASE+ PPA/32│
       │     │  └───────┬──────────┘
       │     │          │
       │     │  ┌───────▼──────────┐
       │     └─►│ NEXT STEP =      │
       └───────►│ NEXT STEP-BASE   │
                └───────┬──────────┘
```

**BIG VALVE FLAG SET ?** — NO / YES

- OPEN SMALL VALVE
- OPEN BIG VALVE
- DEFLATE TO NEXT STEP
- **BIG VALVE FLAG SET ?** — YES / NO
- TIME = TIME TO DEFLATE
- **TIME/BASE < 8mS/TORR** — YES / NO
- NEXT STEP = NEXT STEP + 8

- SET BIG VALVE FLAG
- MEASURE AND STORE DATA
- **ALL DATA COLLECTED ?** — NO / YES
- ( EXIT DEFLATE ROUTINE )

13

FIG-3